# EUROPEAN PATENT SPECIFICATION

(11) **EP 4 129 355 B1**
(45) Date of publication and mention of the grant of the patent: **04.11.2026**
(21) Application number: 22189036.1
(22) Date of filing: 05.08.2022
(51) Int. Cl.: A61M 1/02, A61M 1/30, A61M 1/34, A61M 1/36, A61M 1/38

(54) **SYSTEMS AND METHODS FOR CONVERTING AN APHERESIS FLUID PROCESSING CIRCUIT TO SINGLE OR DOUBLE NEEDLE MODE**
SYSTEME UND VERFAHREN ZUR UMWANDLUNG EINES APHERESE-FLÜSSIGKEITSBEHANDLUNGSKREISLAUFS IN EINEN EIN- ODER DOPPELNADELMODUS
SYSTÈMES ET PROCÉDÉS DE CONVERSION DE CIRCUIT DE TRAITEMENT DE LIQUIDE D'APHÉRÈSE EN MODE D'AIGUILLE UNIQUE OU DOUBLE

(30) Priority: 06.08.2021 US 202163230473 P; 03.08.2022 US 202217880357
(43) Date of publication of application: 08.02.2023
(73) Proprietor: Fenwal, Inc., Lake Zurich, IL 60047 (US)
(72) Inventor: CARLSON, Angela N., Lake Zurich, 60047 (US); COULTAS, Gregory, Lake Zurich, 60047 (US); NGUYEN, Lan T., Lake Zurich, 60047 (US); SCHALTE, Kevin, Lake Zurich, 60047 (US); BURKETT, Grover, Lake Zurich, 60047 (US)
(74) Representative: Maikowski & Ninnemann Patentanwälte Partnerschaft mbB

(56) References cited:
- US-A- 5 956 023
- US-A1- 2014 165 733
- US-A1- 2014 358 061
- US-B2- 10 919 049
- US-B2- 8 211 049

## Description

### Field of the Disclosure

The present disclosure is directed to apheresis systems and methods (not claimed) for processing a biological fluid such as blood. More particularly, the present disclosure is directed to systems and methods for converting an apheresis fluid processing circuit to a single or double needle configuration. Even more particularly, the present disclosure is directed to systems and methods wherein during an apheresis procedure, a double needle apheresis fluid circuit can be converted to a single needle fluid circuit and a single needle fluid circuit can be converted to a double needle fluid circuit.

### Background

The various blood processing systems now make it possible to collect particular blood constituents, rather than whole blood, from donors or patients or other blood sources. Typically, in such systems, whole blood is drawn from a source, the particular blood component or constituent is removed and collected (and/or subjected to further treatment or processing) and the remaining blood constituents are returned to the source (donor or patient). This process of withdrawing whole blood from a donor or patient, separating components, and returning at least some of the components is commonly referred to as "apheresis." By thus removing only particular constituents, potentially less time is needed for the source's body to return to normal (in the case of a living source), and donations can be made at more frequent intervals than when whole blood is collected. This increases the overall supply of blood constituents, such as plasma and platelets, made available for health care.

Whole blood is typically separated into its constituents through centrifugation (although other separation principles, such as a spinning membrane as described in US Patent No 5,194,145 may also be used.) This requires that the whole blood be passed through a centrifuge after it is withdrawn from, and before it is returned to, the source. To avoid contamination and possible infection of the source, the blood is typically contained within a sealed, sterile fluid flow system or disposable fluid circuit during the entire centrifugation process. The disposable fluid circuit may be equipped with one or more access devices or venipuncture needles for accessing the vascular system of the donor or patient. The venipuncture needles are in fluid communication with the tubing and containers of the disposable fluid circuit or "kit".

Typical blood processing systems thus include a permanent, reusable hardware (centrifuge and centrifuge drive system, pumps, valve actuators, programmable controller, and the like) that spins and pumps the blood, and a disposable fluid circuit that is mounted in cooperation on the reusable hardware. The centrifuge assembly engages and spins a separation chamber of the disposable fluid circuit during an apheresis procedure. The blood, however, makes actual contact only with the disposable fluid processing assembly, which assembly is used only once and then discarded.

As noted above, the disposable fluid circuit may be equipped with one or more needles for accessing the vascular system of the donor or patient. In a "single needle" configuration, blood is withdrawn from, and blood components are returned to the donor or patient through the same needle inserted into the vein of the patient or donor. The draw and return cycles are alternated with drawn blood being temporarily sequestered in an "in-process" container. During a return cycle, blood within the in-process container may be introduced into the separation chamber for processing. Similarly, the fluid circuit may include a "return container" which provides a temporary reservoir for separated fluid components during blood draw of a "single needle" procedure, with the contents of the return container subsequently being returned to the blood source (patient or donor) during a return phase.

In a "double needle" configuration, two needles are provided for drawing blood and returning blood components, respectively. The needles may be inserted into separate veins of the subject. Inasmuch as the withdrawal of whole blood and return of blood components are occurring concurrently when using a double needle fluid circuit, the in-process container and a return container may remain unused in a double needle configuration.

The disposable fluid circuit may be equipped and initially provided in a double needle configuration which can be converted to a single needle configuration, as necessary, to address certain events or disruptions that may occur during an apheresis procedure that may render one of the draw and return lines inoperative (e.g., due to a blockage). In this case, the incapacitated line may be directly connected to the viable line, with the procedure then continuing (in some capacity) with the fluid processing assembly in a "single needle" configuration instead of a "double needle" configuration. An example of a convertible fluid processing system and circuit is provided in US Patent No. 10,919,049,.

While the ability to convert a disposable fluid circuit from a double needle configuration to a single needle allows the apheresis procedure to continue notwithstanding a disruption due to blockage, further modifications to apheresis systems may be desired. For example, it may be desirable to convert a disposable fluid circuit in a single needle configuration to a double needle configuration. In addition, when converting the circuit or kit from a double needle configuration to a single needle configuration, parts of the circuit may include stagnant, unanticoagulated blood that may clot and affect blood flow within the fluid circuit. Accordingly, it would be desirable for the controller-based system to flush or otherwise remove any such stagnant and unanticoagulated blood. Inasmuch as the conversion of the apheresis procedure from a single needle to double needle procedure or from a double needle to a single needle procedure will often require a pause in the procedure followed by a resumption of the procedure, it would be desirable to have a system that can verify that the conversion has been correctly completed and that the procedure may resume in its new configuration. Finally, it would be desirable to have a system that tracks multiple parameters of the apheresis procedure and initializes the appropriate variable in the converted single needle or double needle procedure.

### Summary

Insofar as the term embodiment or aspect or alternative is used in the following, or features are presented as being optional, this should be interpreted in such a way that the only protection sought is that of the invention claimed and defined in the appended claims.

According to the invention, a system for converting a disposable fluid circuit from a double needle configuration to a single needle configuration and vice versa during an apheresis procedure is provided.

The present disclosure provides for a system for performing an apheresis procedure. The system includes a reusable, fluid processing device, wherein the device includes valves for opening and closing flow paths of a fluid circuit, one or more pumps for moving blood through the flow paths of a fluid circuit and a controller. The system also includes a disposable fluid circuit mountable onto the device, the circuit including a separation chamber, a draw line including a fluid source access device (needle) wherein the draw line is in flow communication with the chamber, and a return line including a fluid source access device (needle) wherein the draw line is in flow communication with the separation chamber. A controller is included and is configured to (i) provide instructions for converting the disposable fluid circuit from a double needle configuration to a single needle configuration and/or (ii) provide instructions for converting said disposable fluid circuit from a single needle configuration to a double needle configuration, and (iii) perform one or more checks to verify that the apheresis procedure may continue in the converted configuration.

Said system can be used for conducting a method for performing an apheresis procedure. The method includes establishing fluid communication with the vascular system of a donor or patient by inserting one or two needles of a disposable fluid circuit into said one or more veins of said donor or patient; initiating an apheresis procedure by withdrawing blood through a needle of the disposable fluid circuit; pausing the apheresis procedure; reconfiguring the fluid circuit by changing the number of needles in fluid communication with the vascular system of the donor or patient; and resuming the apheresis procedure with said disposable fluid circuit in said reconfigured state.

The systems and methods described herein find application in apheresis procedures generally as well as in apheresis procedures which include or are combined with further treatment of a collected blood component such as the photoactivation treatment of mononuclear cells, commonly referred to as "photopheresis."

### Brief Description of the Drawings

Figure 1 is a perspective view of an apheresis system in accordance with the present disclosure;
Figure 2 is an enlarged perspective view of the front panel the apheresis system of Fig. 1 with a disposable fluid circuit mounted thereon;
Figure 3 is a schematic view of the apheresis system of Figs. 1 and 2 in combination with an irradiation device and a patient embodying an apheresis/photopheresis system in accordance with the present disclosure;
Figure 4 is a schematic view of disposable fluid circuit for use with the apheresis/photopheresis system of Fig. 3;
Figure 5 is a partial view of the disposable fluid circuit of Fig. 4 wherein the circuit has been reconfigured from a double needle configuration to a single needle configuration;
Figure 6 is a flow-chart showing the automated and operator-initiated steps during a conversion of a fluid circuit from a double needle configuration to a single needle configuration; and
Figure 7 is a flow chart showing the automated and operator-initiated steps during a conversion of a fluid circuit from a single needle configuration to a double needle configuration.

### Detailed Description of the Embodiments

A more detailed description of the systems and methods in accordance with the present disclosure is set forth below. It should be understood that the description below of specific devices and methods is intended to be exemplary, and not exhaustive of all possible variations or applications. Thus, the scope of the disclosure is not intended to be limiting and should be understood to encompass variations or embodiments that would occur to persons of ordinary skill.

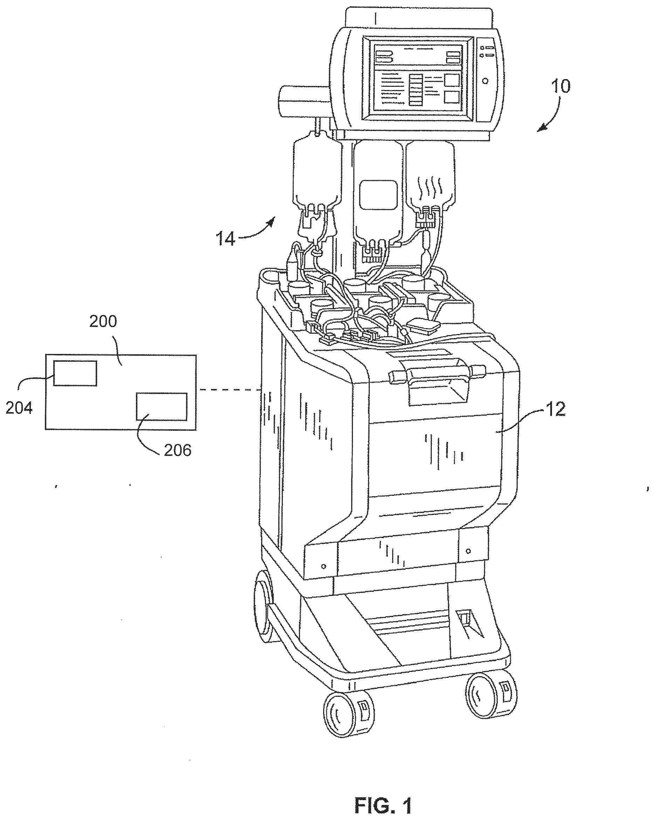

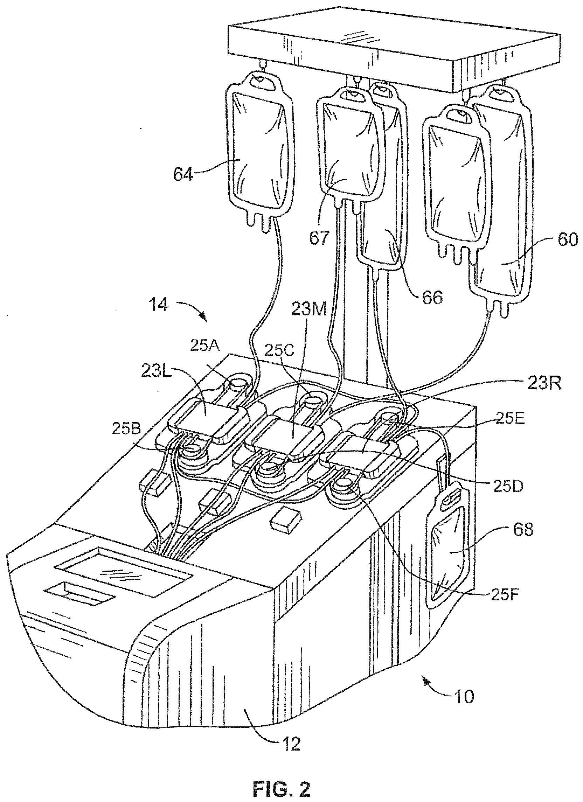

Figs. 1 and 2 show a representative apheresis system 10 useful in the separation of whole blood and collection of blood components. The system 10 includes a reusable hardware component 12 and a disposable fluid circuit or kit 14 that is mounted thereon. In one embodiment, the separation principle used by the separator 12 is based on centrifugation, but an automated separator based on a different separation principle may also be used.

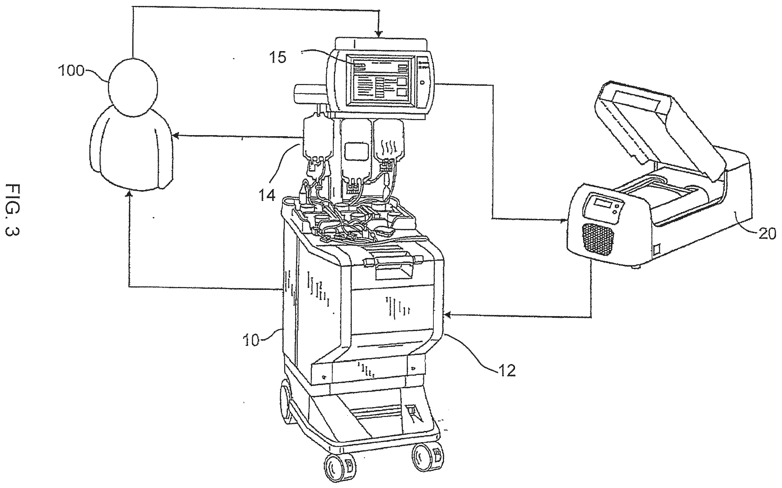

When used to perform photopheresis, an irradiation component 20 housed separately from hardware component 12 is used in conjunction with the separation system 10, as shown schematically in Fig. 3 and described in US 2013/0197419. Although separately housed and independent devices, it is preferable that separation device/component 12 and irradiation device 20 be located adjacent to each other. However, it will be appreciated that the methods described herein may also be used with devices having integrated separation and irradiation components.

In accordance with the systems and methods described herein, a patient (or donor) is connected to disposable fluid circuit 14. As noted above, the fluid circuit 14 provides a sterile closed pathway between the separation component 12 and the irradiation component 20.

In accordance with the present disclosure, whole blood is withdrawn from the patient (or donor) and introduced into the separation component 12, where the whole blood is separated to provide a target cell population, which in the context of photopheresis may be mononuclear cells. Other components separated from the whole blood, such as red blood cells and platelets may be returned to the patient or collected in pre-attached containers of the blood processing set. The separated target cell population, e.g., mononuclear cells, may be prepared for and subjected to a photoactivation treatment.

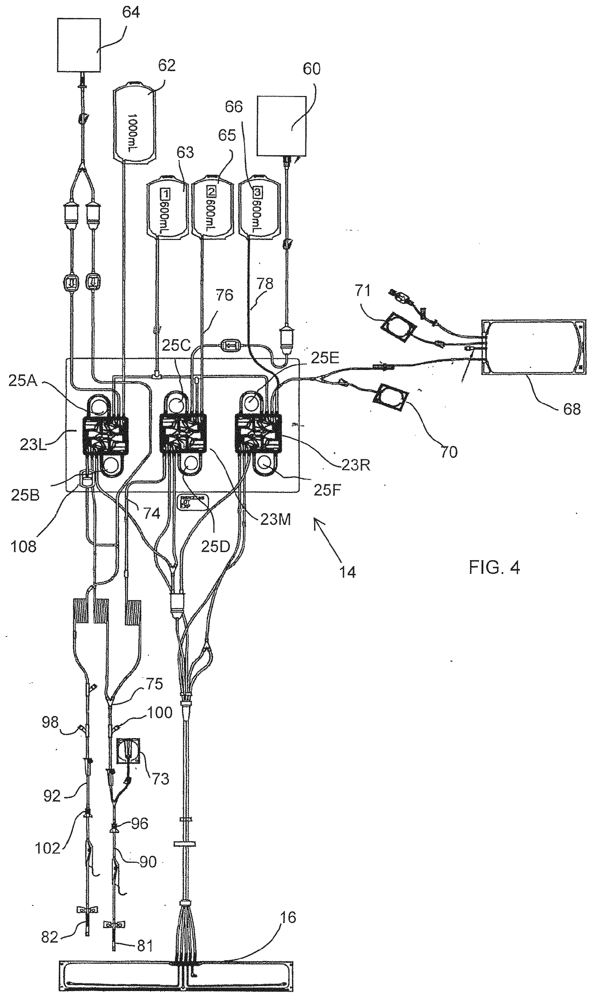

Apparatus useful in the collection of mononuclear cells, and providing the separation component 12 of Fig. 1, include the Amicus^{®} Separator made and sold by Fenwal, Inc., of Lake Zurich, Illinois, which is an affiliate of Fresenius Kabi AG of Bad Homburg, Germany. Mononuclear cell collections using a device such as the Amicus^{®} are described in greater detail in U.S. Patent No. 6,027,657. The fluid circuit (Fig. 4) includes a blood processing container 16 defining a separation chamber suitable for separating and harvesting mononuclear cells (MNC) from whole blood.

As shown in Fig. 2, disposable processing set or fluid circuit 14 (which includes container 16) is mounted on the front panel of the separation component 12. The fluid circuit 14 includes a plurality of processing fluid flow cassettes 23L, 23M and 23R with tubing loops for association with peristaltic pumps 25A-25F on the separation component 12. Fluid circuit 14 also includes a network of tubing and pre-connected containers for establishing flow communication with the patient and for processing and collecting fluids and blood and blood components, as shown in greater detail in Fig. 4 and described below.

As further seen in Fig. 4, the disposable processing set 14 may include a container 60 for supplying anticoagulant, waste container 62 for collecting waste from one or more steps in the process for treating mononuclear cells, container 63 for temporarily holding components to be returned to the donor or patient, container 64 for holding a crystalloid solution, such as saline, or other wash or resuspension medium, in-process container 65 for temporarily holding blood components to be processed, container 66 for collecting plasma, container 68 for collecting the desired blood component which, in a photopheresis system and method may be mononuclear cells and, optionally, a container (not shown) for holding photoactivation agent. In a photopheresis procedure, container 68 may also serve as the illumination container and is preferably pre-attached to disposable set 14. Alternatively, container 68 may be attached to set 14 by known sterile connection techniques, such as sterile docking or the like. Disposable fluid circuit 14 may further include sample pouches 70 and 71 for collecting samples of the collected component (such as mononuclear cells) before and after photoactivation. Fluid circuit 14 may also include sampling pouch 73 for the pre-donation sampling of whole blood.

With further reference to Fig. 4, fluid circuit includes draw line 72, an anticoagulant (AC) line 74 for delivering AC from container 60. Anticoagulant line 74 joins draw line 90 at branch member 75. Fluid circuit 14 further includes a red blood cell (RBC) line 76 for conveying red blood cells from the separation chamber of container 16 to container 65, a platelet-poor plasma (PPP) line 78 for conveying PPP from separation chamber 16 to container 66 and line 80 for conveying mononuclear cells to and from separation chamber 16 and collection/illumination container 68.

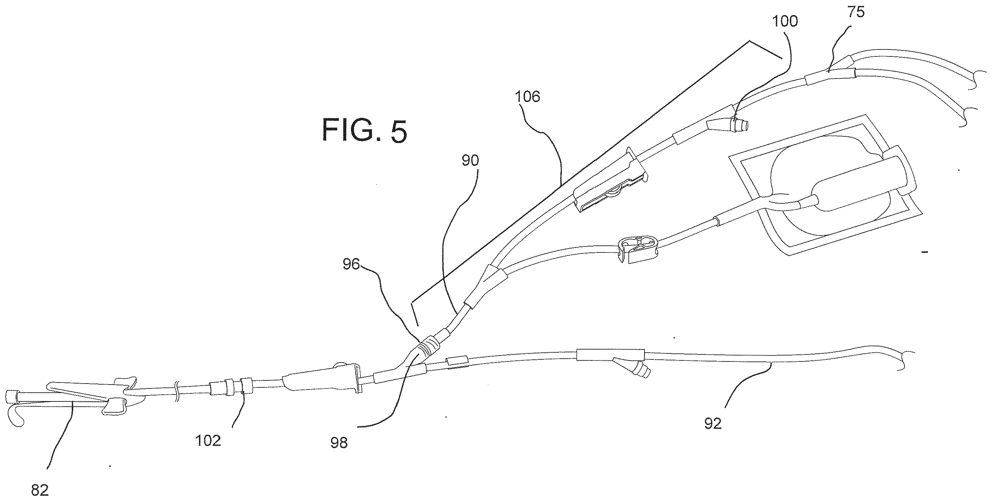

The blood processing set also includes one or more venipuncture needle(s) for accessing the vascular system of the patient. As shown in Fig. 4, fluid circuit 14 includes inlet needle 81 and return needle 82. In accordance with the present disclosure, disposable fluid circuit 14 may initially be provided in a double needle configuration, as shown. In the event that access to draw line 90 has become impaired or rendered inoperative, draw line 90 may be directly connected to the return line 92 for continued processing. In that regard, draw line 90 and return line 92 are provided with mating connectors 96 and 98 that are directly connected to convert the fluid circuit 14 from a "double needle" configuration to a "single needle" configuration, as shown in greater detail in Fig. 5, in which the return line 92 is responsible for both fluid draw and return. In one embodiment, connector 96 of the draw line 90 is configured as a male luer, while the connector 98 on the return line 92 is configured as a female luer, but the exact configuration of the mating connectors 96 and 98 may vary without departing from the scope of the present disclosure.

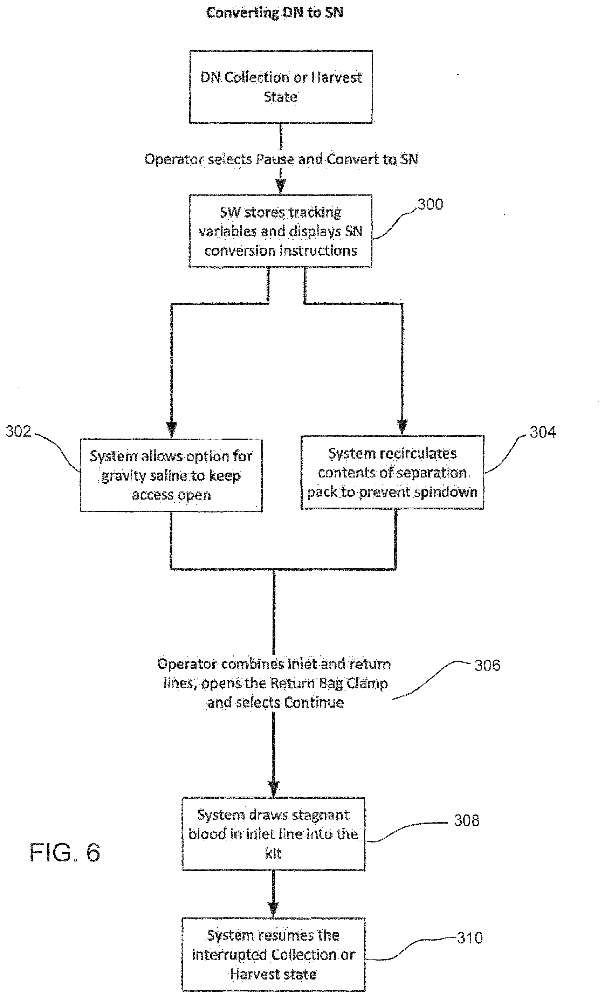

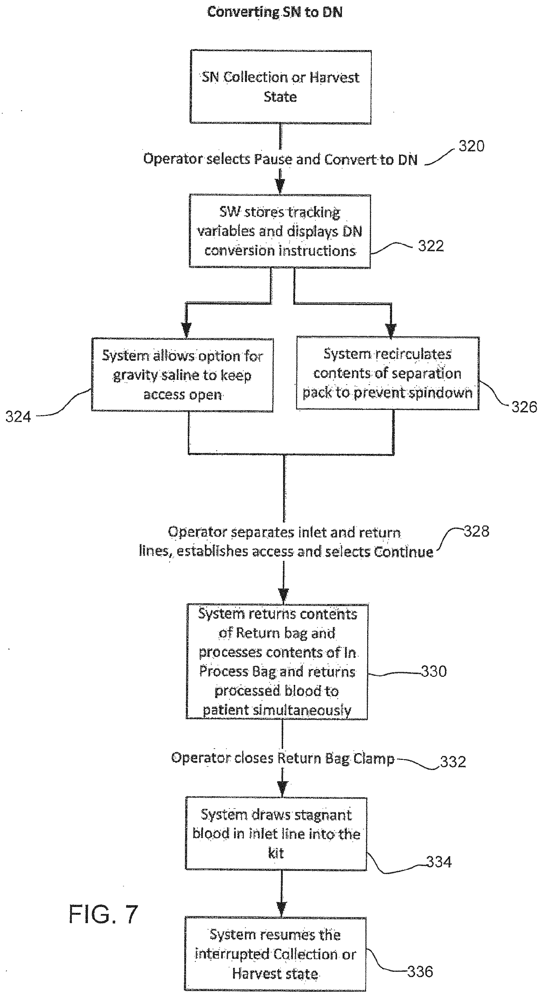

When converting fluid circuit 14 from a double needle configuration to single needle configuration such that return line 92 is responsible for both fluid draw and return (i.e., where the luer connector on the draw line 90 is connected to the luer on the return line 92), care must be taken to ensure that unanticoagulated and potentially stagnant blood in the impaired draw line 90 does not clot and block the flow paths of the fluid circuit 14. Accordingly, soon if not immediately after conversion from a double needle configuration conversion to a single needle configuration (or from a single needle configuration to a double needle configuration) any stagnant, unanticoagulated in draw line 90 may be drawn (pumped by one or more pumps, such as pumps 25A and 25C, under the direction of controller 200) into the disposable fluid circuit, and directed through return line filter 108 to remove any potential clots that may have formed so as to avoid blockage in the centrifuge lines and returned to patient. As noted above, this "one time" post-conversion clearing of stagnant, unanticoagulated blood is preferably performed immediately after conversion from one configuration to another configuration (see also, Figs. 6 and 7).

In addition to the foregoing, where a double needle configuration has been converted to a single needle configuration, additional and repeating "clearing" of stagnant, unanticoagulated blood may be desired. For example, as seen in Fig. 5, blood residing in the portion 106 of draw line 90 (now connected to return line 92) below branch member 75 is unlikely to be anticoagulated and is stagnant during the return cycle, and may become coagulated if the return cycle is long in duration. In accordance with the method and system described herein, stagnant blood residing in portion 106 of the fluid circuit may be cleared prior to each return cycle, as described below.

In one embodiment (and with reference to Figs. 4 and 5), blood components residing in return container 63 may be pumped by pump 25A, under the direction of controller 200, through the draw line 90 and displace any potentially stagnant blood in circuit portion 106. Alternatively, saline or other crystalloid solution may be pumped by one or more pumps, under the direction of controller 200, through draw line 90 to likewise displace any potentially stagnant blood residing in circuit portion 106, although pumping fluid from return container 63 may be preferred inasmuch as this method uses the patient's own blood instead of saline of which an excess amount can be harmful to the patient. While pumping either return fluid or saline allows for better control over volume displacement, the stagnant blood in fluid circuit portion 106 may also be cleared by gravity draining return fluid or saline from containers 63 or 64, respectively. The unanticoagulated blood may be displaced to a point where connectors 96 and 98 are joined (see Fig. 5) to ensure that it is returned during the next return cycle-., (Fig. 4)

In an alternative embodiment, where return line 92 has been rendered inoperative, it may be directly connected to the draw line 90 for continued processing in a manner that is similar to the double needle to single needle conversion described above. As described above, the draw line 90 and return line 92 are provided with mating connectors 100 and 102 that are directly connected to convert the fluid circuit 14 from a "double needle" configuration to a "single needle" configuration in which now the draw line 90 is responsible for both fluid draw and return. Connector 100 of the draw line 90 may be configured as a female luer, while the connector 102 on the return line 92 is configured as a male luer, but the exact configuration of the mating connectors 100 and 102 may vary without departing from the scope of the present disclosure.

In a further alternative embodiment, fluid circuit 14 may be converted from a single needle configuration to a double needle configuration. In this regard, a previously established luer connection whereby a double needle configuration had been converted to a single needle configuration may be undone and the draw and return lines 90,92 separated. Male luer 96 on draw line 90 or male luer 102 on return line 92 may be connected to a new venipuncture needle 81 or 82 assembly to convert the fluid circuit (back) to a double needle configuration.

Fluid flow through fluid circuit 14 is preferably driven, controlled, and adjusted by a microprocessor-based controller 200 in cooperation with the valves, pumps, weight scales and sensors of separation component 12 and fluid circuit 14, the details of which are described in the previously mentioned U.S. Patent No. 6,027,657. As discussed above, controller 200 may also initiate pump rotation to pump return fluid or saline to clear stagnant blood residing in the fluid circuit 14 and fluid circuit portion 106 specifically.

Controller 200 (schematically shown in Fig. 1) may include a microprocessor 204 (which may include multiple physical and/or virtual processors). According to other embodiments, the controller 200 may include one or more electrical circuits designed to carry out the actions described herein. In an embodiment, controller 200 may include a microprocessor and other circuits or circuitry. In addition, the controller 200 may include one or more memories 206. The instructions by which the microprocessor is programmed may be stored on the memory 206 associated with the microprocessor 204, which memory/memories 206 may include one or more tangible non-transitory computer readable memories, having computer executable instructions stored thereon, which when executed by the microprocessor 204, may cause the microprocessors 204 and associated software to carry out one or more actions as described herein.

The processing following conversion from a "double needle" configuration to a "single needle" configuration or conversion from a "single needle" to a "double needle" configuration may vary depending on a number of factors, including the current procedure state or phase at the time of conversion. The controller of the fluid processing system 10 may monitor or track at least one or more of the amount of whole blood processed, the amount of whole blood withdrawn, the amount of whole blood returned, the current procedure state (such as, but not limited to, an initial processing state, a plasma collection state, a MNC collection state, a red blood cell collection state), the current extracorporeal volume, current red blood cell volume, the current fluid balance and use that information to determine how to proceed once processing is unpaused/continued after conversion from one configuration to another configuration. The controller may also initialize the appropriate variables unique to either a single needle procedure or a double needle procedure in the new single needle or double needle configuration. Depending on the circumstances, the controller may determine that it is appropriate to continue the procedure state or phase that was being executed at the time of termination. If the controller instead determines that it would be inappropriate to continue with the procedure state or phase that was being executed at the time of termination, then it may instead initiate a different procedure state or phase, which may be the phase or procedure state immediately following the phase or procedure state that was being executed at the time of termination, a variation of such succeeding phase or procedure state, or some other phase or procedure state

Conversion from a double needle configuration to a single needle configuration may be initiated at any time during the collection or harvesting of blood components. The operator may begin the conversion process by selecting "Pause and Convert to SN" on the graphical user interface/touch screen 15 of hardware unit 12 (Fig. 1). Controller 200 stores the tracking variables and displays "SN" conversion instructions on the user interface 15 or other computer screen, as shown in step 300 of Fig. 6. The system allows for gravity delivery of saline to keep access to the patient open and facilitate the return of blood components and/or recirculate contents in separation chamber 16 to prevent spindown as shown in steps 302 and 304. "Spindown" refers to a condition of the separator whereby flow through the separator (e.g., centrifuge) during the pause in the procedure to allow for conversion results is diminished flow and consequently, an automatic "spinning down" of the centrifuge so as to prevent heating of the blood. To prevent the automatic spindown, a small volume of blood is recirculated slowly through the centrifuge. The operator then performs the manual conversion by combining the draw and return lines, opens the Return container clamp and selects "Continue" on the screen (step 306). The system then draws stagnant blood into the kit (step 308), as described above, and resumes collection/harvesting in its new configuration.
Conversion from a single needle configuration to a double needle configuration is shown in Fig. 7. As in the double needle to single needle conversion, the operator selects Pause and Convert to DN on the graphical user interface or computer screen (step 320). Controller 200 stores the tracking variables and displays DN conversion instructions on the user interface 15 or other computer screen, as shown in step 322. The system allows for gravity delivery of saline to keep access open and/or recirculates contents in separation chamber 16 to prevent spindown as shown in steps 324 and 326. The operator then performs the manual conversion of the single need kit to a double needle kit, as previously described (step 328). Under the direction of the controller, the system then returns the previously collected (while in the single needle configuration) contents of the return container 63 and processes the contents of in-process container 65. Processed blood components are then returned to the patient simultaneously with the return fluid (step 330). The operator then closes the clamp to return container 63 (step 332). Under the direction of the controller, the system then draws stagnant blood in the inlet line into the kit (step 334) and the system resumes the collection and harvesting of components.

## Claims

1. A system (10) for converting a disposable fluid circuit from a double needle configuration to a single needle configuration and vice versa during an apheresis procedure comprising:
a. a reusable, fluid processing device (12), said device comprising valves for opening and closing flow paths of a fluid circuit, one or more pumps (25A-F) for moving blood through flow paths of a fluid circuit and a controller (200);
b. a disposable fluid circuit (14) mountable onto said device (12), said circuit comprising a separation chamber (16), a draw line (90) including a fluid source access device (81) wherein said draw line (90) is in flow communication with said chamber (16), and a return line (92) including a fluid source access device (82) and a filter (108), wherein said return line (92) is in flow communication with said chamber (16); the draw line (90) and return line (92) each comprising mating connectors ( 96, 100; 98, 102) configured to be directly and removably connected to each other such that the disposable fluid circuit can be converted from a double needle configuration to a single needle configuration and vice versa,
c. wherein said controller (200) is configured to (i) provide instructions for converting said disposable fluid circuit (14) from a double needle configuration to a single needle configuration and/or (ii) provide instructions for converting said disposable fluid circuit from a single needle configuration to a double needle configuration (iii) perform one or more checks to verify that the apheresis procedure may continue with the disposable fluid circuit (14) in the converted configuration, and (iv) activate one or more pumps (25A,C) to draw any stagnant, unanticoagulated blood in the draw line (90) into the disposable fluid circuit (14) and through the filter (108) on the return line (92) after said conversion from a double needle configuration to a single needle configuration and vice versa.

2. The system of Claim 1 wherein said controller (200) is further configured to monitor one or more of the following procedure parameters: (a) volume of whole blood processed (b) volume of whole blood drawn from a blood source (c) volume of whole blood returned (d) current procedure state (e) current extracorporeal volume (f) current red blood cell volume removed from source (g) current fluid balance of said source.

3. The system of any one of Claims 1 through 2 wherein said controller (200) is configured to pause said apheresis procedure during conversion from one configuration to another configuration and resume said apheresis procedure once said conversion has been completed.

4. The system of any one of Claims 1 through 3 wherein said disposable fluid circuit further comprises an in-process container (65) for holding blood or a blood component to be processed and a return container (63) for holding a blood component to be returned to a blood source.

5. The system of Claim 4 wherein said controller (200) is further configured to initiate processing of said blood in said in-process container (65) and initiate return of said blood component in said return container (63) to said source.

6. The system of Claim 5 wherein said controller is configured to initiate processing of said blood in said in-process container (65) and initiate return of said blood component in said return container (63) to said source after converting said disposable fluid circuit from a single needle configuration to a double needle configuration.

7. The system of any one of Claims 1 through 6 wherein said draw line (90) includes an access port for receiving said return line (92).

8. The system of any one of Claims 1 through 7 wherein said return line (92) includes an access port for receiving said draw line (90).

9. The system of any one of Claims 1 through 8 further comprising an irradiation device (20).

10. The system of Claim 9 wherein said irradiation device (20) is housed separately from said fluid processing device.

11. The system of any one of Claims 1 through 10 wherein said controller (200) is configured to activate one or more pumps and to pump saline or a blood component to displace unanticoagulated blood within said fluid circuit.

12. The system of Claim 11 wherein said controller (200) is configured to activate said one or more pumps to displace said unanticoagulated blood to a selected location of the fluid circuit.

## Patentansprüche

1. System (10) zum Umstellen eines Einweg-Fluidkreislaufs von einer Zweinadelkonfiguration auf eine Einnadelkonfiguration und umgekehrt während eines Aphereseverfahrens, umfassend:
a. eine wiederverwendbare Fluidverarbeitungsvorrichtung (12), wobei die genannte Vorrichtung Ventile zum Öffnen und Schließen von Strömungswegen eines Fluidkreislaufs, eine oder mehrere Pumpen (25A-F) zum Fördern von Blut durch Strömungswege eines Fluidkreislaufs und eine Steuerung (200) umfasst;
b. einen an der genannten Vorrichtung (12) montierbaren Einweg-Fluidkreislauf (14), wobei der genannte Kreislauf eine Trennkammer (16), eine Entnahmeleitung (90), die eine Zugangsvorrichtung zu einer Fluidquelle (81) umfasst, wobei die genannte Entnahmeleitung (90) mit der genannten Kammer (16) in Fluidverbindung steht, und eine Rückführleitung (92), die eine Zugangsvorrichtung zu einer Fluidquelle (82) und einen Filter (108) umfasst, wobei die genannte Rückführleitung (92) mit der genannten Kammer (16) in Fluidverbindung steht; wobei die Entnahmeleitung (90) und die Rückführleitung (92) jeweils zusammenpassende Verbinder (96, 100; 98, 102) umfassen, die dazu eingerichtet sind, direkt und lösbar miteinander verbunden zu werden, sodass der Einweg-Fluidkreislauf von einer Zweinadelkonfiguration auf eine Einnadelkonfiguration und umgekehrt umgestellt werden kann,
c. wobei die genannte Steuerung (200) dazu eingerichtet ist, (i) Anweisungen zum Umstellen des genannten Einweg-Fluidkreislaufs (14) von einer Zweinadelkonfiguration auf eine Einnadelkonfiguration bereitzustellen und/oder (ii) Anweisungen zum Umstellen des genannten Einweg-Fluidkreislaufs von einer Einnadelkonfiguration auf eine Zweinadelkonfiguration bereitzustellen (iii) eine oder mehrere Prüfungen durchzuführen, um zu verifizieren, dass das Aphereseverfahren mit dem Einweg-Fluidkreislauf (14) in der umgestellten Konfiguration fortgesetzt werden kann, und (iv) eine oder mehrere Pumpen (25A,C) zu aktivieren, um jegliches in der Entnahmeleitung (90) stagnierende, nicht antikoagulierte Blut in den Einweg-Fluidkreislauf (14) und durch den Filter (108) an der Rückführleitung (92) zu fördern, nachdem die genannte Umstellung von einer Zweinadelkonfiguration auf eine Einnadelkonfiguration und umgekehrt erfolgt ist.

2. System nach Anspruch 1, wobei die genannte Steuerung (200) ferner dazu eingerichtet ist, einen oder mehrere der folgenden Verfahrensparameter zu überwachen: (a) Volumen des verarbeiteten Vollbluts (b) Volumen des einer Blutquelle entnommenen Vollbluts (c) Volumen des zurückgeführten Vollbluts (d) aktueller Verfahrenszustand (e) aktuelles extrakorporales Volumen (f) aktuelles, der Quelle entnommenes Erythrozytenvolumen (g) aktuelle Fluidbilanz der genannten Quelle.

3. System nach einem der Ansprüche 1 bis 2, wobei die genannte Steuerung (200) dazu eingerichtet ist, das genannte Aphereseverfahren während der Umstellung von einer Konfiguration auf eine andere Konfiguration zu pausieren und das genannte Aphereseverfahren wiederaufzunehmen, sobald die genannte Umstellung abgeschlossen ist.

4. System nach einem der Ansprüche 1 bis 3, wobei der genannte Einweg-Fluidkreislauf ferner einen Prozessbehälter (65) zum Aufnehmen von zu verarbeitendem Blut oder einer zu verarbeitenden Blutkomponente und einen Rückführbehälter (63) zum Aufnehmen einer an eine Blutquelle zurückzuführenden Blutkomponente umfasst.

5. System nach Anspruch 4, wobei die genannte Steuerung (200) ferner dazu eingerichtet ist, die Verarbeitung des genannten Bluts in dem genannten Prozessbehälter (65) einzuleiten und die Rückführung der genannten Blutkomponente in dem genannten Rückführbehälter (63) zu der genannten Quelle einzuleiten.

6. System nach Anspruch 5, wobei die genannte Steuerung dazu eingerichtet ist, die Verarbeitung des genannten Bluts in dem genannten Prozessbehälter (65) einzuleiten und die Rückführung der genannten Blutkomponente in dem genannten Rückführbehälter (63) zu der genannten Quelle einzuleiten, nachdem der genannte Einweg-Fluidkreislauf von einer Einnadelkonfiguration auf eine Zweinadelkonfiguration umgestellt worden ist.

7. System nach einem der Ansprüche 1 bis 6, wobei die genannte Entnahmeleitung (90) einen Zugangsport zur Aufnahme der genannten Rückführleitung (92) umfasst.

8. System nach einem der Ansprüche 1 bis 7, wobei die genannte Rückführleitung (92) einen Zugangsport zur Aufnahme der genannten Entnahmeleitung (90) umfasst.

9. System nach einem der Ansprüche 1 bis 8, ferner umfassend eine Bestrahlungsvorrichtung (20).

10. System nach Anspruch 9, wobei die genannte Bestrahlungsvorrichtung (20) getrennt von der genannten Fluidverarbeitungsvorrichtung untergebracht ist.

11. System nach einem der Ansprüche 1 bis 10, wobei die genannte Steuerung (200) dazu eingerichtet ist, eine oder mehrere Pumpen zu aktivieren und Kochsalzlösung oder eine Blutkomponente zu pumpen, um nicht antikoaguliertes Blut innerhalb des genannten Fluidkreislaufs zu verdrängen.

12. System nach Anspruch 11, wobei die genannte Steuerung (200) dazu eingerichtet ist, die genannte eine Pumpe oder die genannten mehreren Pumpen zu aktivieren, um das genannte nicht antikoagulierte Blut an eine ausgewählte Stelle des Fluidkreislaufs zu verdrängen.

## Revendications

1. Système (10) destiné à convertir un circuit de fluide jetable d'une configuration à deux aiguilles en une configuration à aiguille unique et inversement au cours d'une procédure d'aphérèse, comprenant :
a. un dispositif réutilisable de traitement de fluide (12), ledit dispositif comprenant des vannes destinées à ouvrir et à fermer des voies d'écoulement d'un circuit de fluide, une ou plusieurs pompes (25A-F) destinées à faire circuler du sang à travers des voies d'écoulement d'un circuit de fluide et un contrôleur (200) ;
b. un circuit de fluide jetable (14) pouvant être monté sur ledit dispositif (12), ledit circuit comprenant une chambre de séparation (16), une ligne de prélèvement (90) comprenant un dispositif d'accès à une source de fluide (81), ladite ligne de prélèvement (90) étant en communication fluidique avec ladite chambre (16), et une ligne de retour (92) comprenant un dispositif d'accès à une source de fluide (82) et un filtre (108), ladite ligne de retour (92) étant en communication fluidique avec ladite chambre (16) ; la ligne de prélèvement (90) et la ligne de retour (92) comprenant chacune des connecteurs complémentaires (96, 100 ; 98, 102) configurés pour être raccordés directement et de manière amovible les uns aux autres, de telle sorte que le circuit de fluide jetable puisse être converti d'une configuration à deux aiguilles en une configuration à aiguille unique et inversement,
c. dans lequel ledit contrôleur (200) est configuré pour (i) fournir des instructions en vue de convertir ledit circuit de fluide jetable (14) d'une configuration à deux aiguilles en une configuration à aiguille unique et/ou (ii) fournir des instructions en vue de convertir ledit circuit de fluide jetable d'une configuration à aiguille unique en une configuration à deux aiguilles (iii) effectuer une ou plusieurs vérifications afin de vérifier que la procédure d'aphérèse peut se poursuivre avec le circuit de fluide jetable (14) dans la configuration convertie, et (iv) activer une ou plusieurs pompes (25A,C) afin d'aspirer tout sang stagnant, non anticoagulé, se trouvant dans la ligne de prélèvement (90), dans le circuit de fluide jetable (14) et à travers le filtre (108) sur la ligne de retour (92) après ladite conversion d'une configuration à deux aiguilles en une configuration à aiguille unique et inversement.

2. Système selon la revendication 1, dans lequel ledit contrôleur (200) est en outre configuré pour surveiller un ou plusieurs des paramètres de procédure suivants : (a) volume de sang total traité (b) volume de sang total prélevé d'une source de sang (c) volume de sang total retourné (d) état actuel de la procédure (e) volume extracorporel actuel (f) volume actuel de globules rouges prélevé de la source (g) bilan de fluide actuel de ladite source.

3. Système selon l'une quelconque des revendications 1 à 2, dans lequel ledit contrôleur (200) est configuré pour mettre en pause ladite procédure d'aphérèse pendant la conversion d'une configuration en une autre configuration et reprendre ladite procédure d'aphérèse une fois ladite conversion achevée.

4. Système selon l'une quelconque des revendications 1 à 3, dans lequel ledit circuit de fluide jetable comprend en outre un récipient de traitement (65) destiné à contenir du sang ou un composant sanguin à traiter et un récipient de retour (63) destiné à contenir un composant sanguin à retourner à une source de sang.

5. Système selon la revendication 4, dans lequel ledit contrôleur (200) est en outre configuré pour initier le traitement dudit sang dans ledit récipient de traitement (65) et initier le retour dudit composant sanguin dans ledit récipient de retour (63) vers ladite source.

6. Système selon la revendication 5, dans lequel ledit contrôleur est configuré pour initier le traitement dudit sang dans ledit récipient de traitement (65) et initier le retour dudit composant sanguin dans ledit récipient de retour (63) vers ladite source après avoir converti ledit circuit de fluide jetable d'une configuration à aiguille unique en une configuration à deux aiguilles.

7. Système selon l'une quelconque des revendications 1 à 6, dans lequel ladite ligne de prélèvement (90) comprend un port d'accès destiné à recevoir ladite ligne de retour (92).

8. Système selon l'une quelconque des revendications 1 à 7, dans lequel ladite ligne de retour (92) comprend un port d'accès destiné à recevoir ladite ligne de prélèvement (90).

9. Système selon l'une quelconque des revendications 1 à 8, comprenant en outre un dispositif d'irradiation (20).

10. Système selon la revendication 9, dans lequel ledit dispositif d'irradiation (20) est logé séparément dudit dispositif de traitement de fluide.

11. Système selon l'une quelconque des revendications 1 à 10, dans lequel ledit contrôleur (200) est configuré pour activer une ou plusieurs pompes et pour pomper une solution saline ou un composant sanguin afin de déplacer du sang non anticoagulé à l'intérieur dudit circuit de fluide.

12. Système selon la revendication 11, dans lequel ledit contrôleur (200) est configuré pour activer lesdites une ou plusieurs pompes afin de déplacer ledit sang non anticoagulé vers un emplacement sélectionné du circuit de fluide.
